# EUROPEAN PATENT SPECIFICATION

(11) **EP 2 162 425 B1**
(45) Date of publication and mention of the grant of the patent: **07.12.2011**
(21) Application number: 08769837.9
(22) Date of filing: 30.05.2008
(51) Int. Cl.: C07C 211/61, H01L 51/00

(54) **BLUE LUMINESCENT MATERIALS**
BLAUE LUMINESZENTE MATERIALIEN
MATÉRIAUX LUMINESCENTS BLEUS

(30) Priority: 01.06.2007 US 941481 P
(43) Date of publication of application: 17.03.2010
(73) Proprietor: E. I. Du Pont de Nemours and Company, Wilmington, DE 19898 (US)
(72) Inventor: HERRON, Norman, Newark, DE 19711 (US); LECLOUX, Daniel, David, Wilmington, DE 19803 (US); GUIDRY, Mark, A., New Castle, DE 19720 (US); NORTH, Victoria, J., Newark, DE 19702 (US); DOBBS, Kerwin, D., Wilmington, DE 19803 (US)
(74) Representative: Towler, Philip Dean
(86) International application number: PCT/US2008/065189
(87) International publication number: WO 2008/150942

(56) References cited:
- WO-A-2005/049546
- WO-A-2007/108666
- US-A1- 2004 209 118
- US-A1- 2005 244 670
- H. W. WEINE ET AL: "Reactions of an o-quinone monoimide with antracenes,phencyclone and 1,3-diphenylisobenzofuran" JOURNAL OF ORGANIC CHEMISTRY., vol. 54, 1989, - 5926 page 5930, XP002497748 USAMERICAN CHEMICAL SOCIETY, WASHINGTON, DC.

## Description

### RELATED APPLICATION DATA

This application claims priority under 35 U.S.C. § 119(e) from U.S. Provisional Application No. 60/941,481 filed on June 1, 2007.

### BACKGROUND INFORMATION

### Field of the Disclosure

This disclosure relates in general to blue luminescent materials and their synthesis.

### Description of the Related Art

Organic electronic devices that emit light, such as light-emitting diodes that make up displays, are present in many different kinds of electronic equipment. In all such devices, an organic active layer is sandwiched between two electrical contact layers. At least one of the electrical contact layers is light-transmitting so that light can pass through the electrical contact layer. The organic active layer emits light through the light-transmitting electrical contact layer upon application of electricity across the electrical contact layers.

It is well known to use organic electroluminescent compounds as the active component in light-emitting diodes. Simple organic molecules, such as anthracene, thiadiazole derivatives, and coumarin derivatives are known to show electroluminescence. In some cases these small molecule materials are present as a dopant in a host material to improve processing and/or electronic properties.

US 2005/0244670 discloses monoamino compounds suitable for use as organoluminescent compounds in electronic devices.

There is a continuing need for new emissive materials, especially for luminescent compounds that are blue-emitting.

### SUMMARY

There is provided a blue luminescent material having Formula I 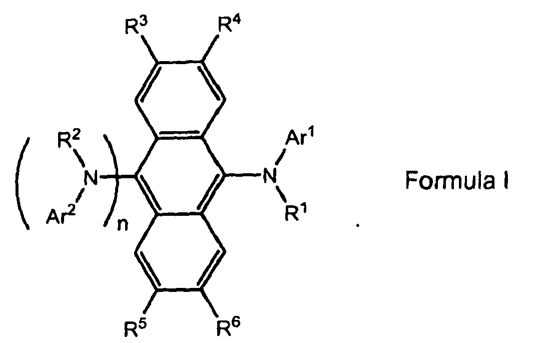 wherein:
Ar1 and Ar2 are the same or different and each is an aryl group having at least two electron-withdrawing substituents selected from the consisting of fluoro, cyano, perfluoroalkyl, nitro, -SO₂R, and combinations thereof, where R is alkyl or perfluoroalky.
   Ar1 and Ar2 are the same or different and each is an aryl group having at least two electron-withdrawing substituents;
   R¹ and R² are the same or different and are selected from the group consisting of alkyl, and aryl;
   R³ R⁴, R⁵ and R⁶ are the same or different and are selected from the group consisting of hydrogen, alkyl, and alkoxy; and
   n is 1.

There is also provided an organic electronic device comprising a first electrical contact, a second electrical contact and a photoactive layer therebetween, the photoactive layer comprising the above blue luminescent material.

The foregoing general description and the following detailed description are exemplary and explanatory only and are not restrictive of the invention, as defined in the appended claims.

### BRIEF DESCRIPTION OF THE DRAWINGS

Embodiments are illustrated in the accompanying figures to improve understanding of concepts as presented herein.

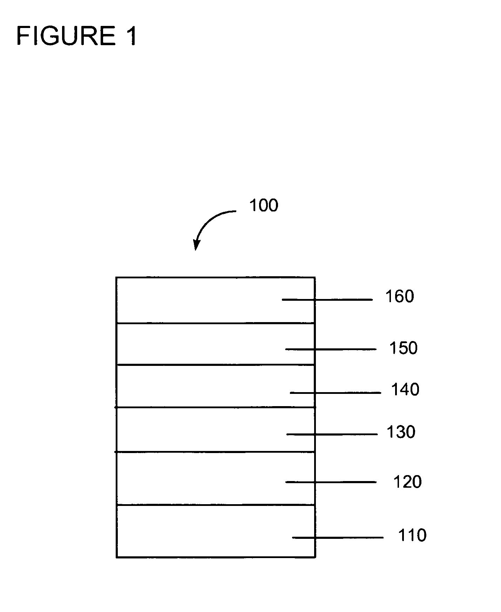

FIG. 1 includes as illustration of an organic light-emitting diode.

Skilled artisans appreciate that objects in the figures are illustrated for simplicity and clarity and have not necessarily been drawn to scale. For example, the dimensions of some of the objects in the figures may be exaggerated relative to other objects to help to improve understanding of embodiments.

### DETAILED DESCRIPTION

Many aspects and embodiments are described herein and are merely exemplary and not limiting. After reading this specification, skilled artisans appreciate that other aspects and embodiments are possible without departing from the scope of the invention.

Other features and benefits of any one or more of the embodiments will be apparent from the following detailed description, and from the claims. The detailed description first addresses Definitions and Clarification of Terms followed by the Blue Luminescent Materials, Synthesis, Devices, and finally Examples.

### 1. Definitions and Clarification of Terms

Before addressing details of embodiments described below, some terms are defined or clarified.

The term "alkoxy" is intended to mean a group having the formula -OR, which is attached via the oxygen, where R is an alkyl.

The term "alkyl" is intended to mean a group derived from an aliphatic hydrocarbon and includes a linear, a branched, or a cyclic group. In some embodiments, an alkyl has from 1-20 carbon atoms.

The term "aromatic compound" is intended to mean an organic compound comprising at least one unsaturated cyclic group having delocalized pi electrons.

The term "aryl" is intended to mean a group derived from an aromatic hydrocarbon having one point of attachment. The term includes groups which have a single ring and those which have multiple rings which can be joined by a single bond or fused together. The term is intended to include heteroaryls. The term "arylene" is intended to mean a group derived from an aromatic hydrocarbon having two points of attachment. In some embodiments, an aryl group has from 3-60 carbon atoms.

The term "binaphthyl" is intended to mean a group having two naphthalene units joined by a single bond. In some embodiments, the binaphthyl group is 1,1-binaphthyl, which is attached at the 3-, 4-, or 5-position; in some embodiments, 1,2-binaphthyl, which is attached at the 3-, 4-, or 5-position on the 1-naphthyl moiety, or the 4- or 5-position on the 2-naphthyl moiety; and in some embodiments, 2,2-binaphthyl, which is attached at the 4- or 5-position.
The term "biphenyl" is intended to mean a group having two phenyl units joined by a single bond. The group can be attached at the 2-, 3-, or 4-position.

The term "cycloalkyl" is intended to mean an alkyl group having one or more ring structures. In some embodiments, a cycloalkyl has from 4-20 carbon atoms.

The term "electron-withdrawing" as it refers to a substituent group is intended to mean a group which decreases the electron density of an aromatic ring.

The term "tertiary alkyl" is intended to mean an alkyl group which includes a tertiary carbon. In some embodiments,

The term "tertiary carbon" is intended to mean a carbon linked to three additional carbons.

All groups may be unsubstituted or substituted. In some embodiments, the substituents are selected from the group consisting of halide, alkyl, alkoxy, aryl, and cyano.

The terms "luminescent material" and "emitter" are intended to mean a material that emits light when activated by an applied voltage (such as in a light-emitting diode or light-emitting electrochemical cell).

The term "blue luminescent material" is intended to mean a material capable of emitting radiation that has an emission maximum at a wavelength in a range of approximately 400-500 nm. In some embodiments, blue light has color coordinates of x = 0.1-0.2, and y = 0.1-0.25, according to the C.I.E. chromaticity scale (Commision Internationale de L'Eclairage, 1931).

The term "layer" is used interchangeably with the term "film" and refers to a coating covering a desired area. The term is not limited by size. The area can be as large as an entire device or as small as a specific functional area such as the actual visual display, or as small as a single sub-pixel. Layers and films can be formed by any conventional deposition technique, including vapor deposition, liquid deposition (continuous and discontinuous techniques), and thermal transfer. Continuous deposition techniques, include but are not limited to, spin coating, gravure coating, curtain coating, dip coating, slot-die coating, spray coating, and continuous nozzle coating. Discontinuous deposition techniques include, but are not limited to, ink jet printing, gravure printing, and screen printing.

The term "organic electronic device" or sometimes just "electronic device" is intended to mean a device including one or more organic semiconductor layers or materials.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components described herein. This is done merely for convenience and to give a general sense of the scope of the invention. This description should be read to include one or at least one and the singular also includes the plural unless it is obvious that it is meant otherwise.

Group numbers corresponding to columns within the Periodic Table of the elements use the "New Notation" convention as seen in the CRC *Handbook of Chemistry and Physics*, 81^{st} Edition (2000-2001).

Unless otherwise defined, all technical and scientific terms used herein have the same meaning as commonly understood by one of ordinary skill in the art to which this invention belongs. Although methods and materials similar or equivalent to those described herein can be used in the practice or testing of embodiments of the present invention, suitable methods and materials are described below. All publications, patent applications, patents, and other references mentioned herein are incorporated by reference in their entirety, unless a particular passage is cited in case of conflict, the present specification, including definitions, will control. In addition, the materials, methods, and examples are illustrative only and not intended to be limiting.

To the extent not described herein, many details regarding specific materials, processing acts, and circuits are conventional and may be found in textbooks and other sources within the organic light-emitting diode display, photodetector, photovoltaic, and semiconductive member arts.

### 2. Blue Luminescent Materials

The new blue luminescent materials described herein have Formula I 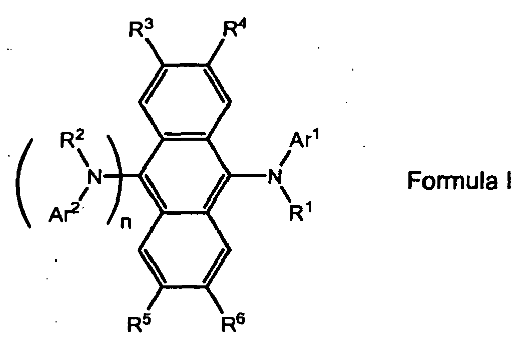 wherein:
Ar1 and Ar2 are the same or different and each is an aryl group having at least two electron-withdrawing substituents;
R¹ and R² are the same or different and are selected from the group consisting of alkyl, and aryl;
R³, R⁴, R⁵ and R⁶ are the same or different and are selected from the group consisting of hydrogen, alkyl, and alkoxy; and
n is 1.

In some embodiments, Ar1 and Ar2 are selected from the group consisting of phenyl, biphenyl, naphthyl, and binaphthyl.

The electron-withdrawing group ("EWG") is selected from the group consisting of fluoro, cyano, perfluoroalkyl, nitro, -SO₂R, where R is alkyl or perfluoroalkyl, and combinations thereof. In some embodiments, the EWG is fluoro. In some embodiments, there are two EWGs on each of Ar1 and Ar2; in some embodiments, 3-5 EWGs. In some embodiments, there is at least one EWG in a position that is ortho to the position bonded to the nitrogen.

In some embodiments, at least one of R¹ and R² is an alkyl. In some embodiments, both R¹ and R² are aryl groups selected from the group consisting of phenyl, biphenyl, naphthyl, and binaphthyl. In some embodiments, the aryl group is a substituted aryl group. In some embodiments, the aryl group has at least one substituent selected from the group consisting of EWG, alkyl and alkoxy.

In some embodiments, R³ = R⁵ = H, and R⁴ = R⁶ = branched alkyl or cycloalkyl. In some embodiments, R³ = R⁶ = branched alkyl or cycloalkyl, and R⁴ = R⁵ = H. In some embodiments, the branched alkyl has 3-8 carbon atoms. In some embodiments, the branched alkyl is selected from the group consisting of isopropyl, 2-butyl, t-butyl, neopentyl, and 2-ethylhexyl groups. In some embodiments, the cycloalkyl group has from 6-20 carbon atoms; in some embodiments, 6-12. Examples of suitable cycloalkyl groups include, but are not limited to, cyclohexyl, 1-methylcyclohexyl, and adamantyl groups.

Examples of blue luminescent materials having Formula I include, but are not limited to, compounds B1 through B8 below:

### Compound B1

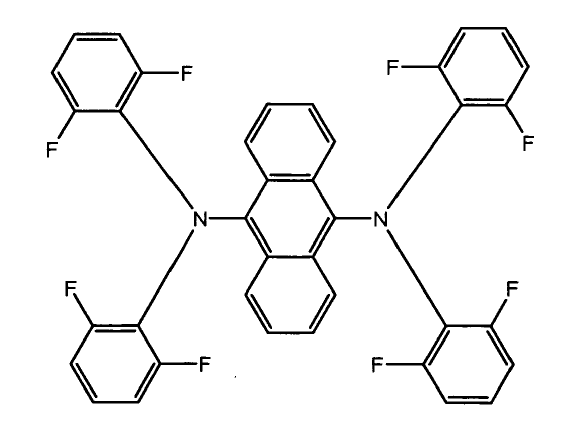

### Compound B2

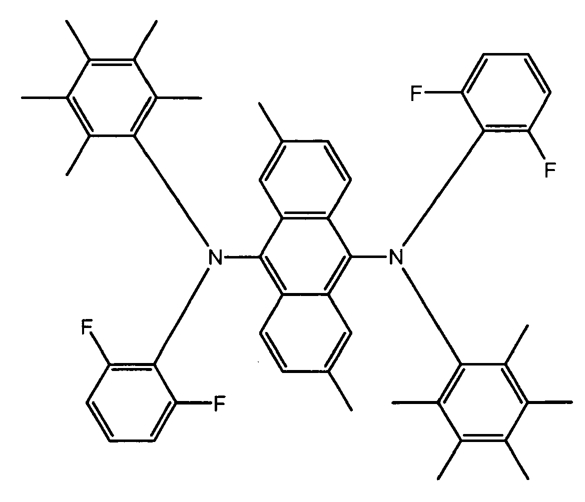

### Compound B3

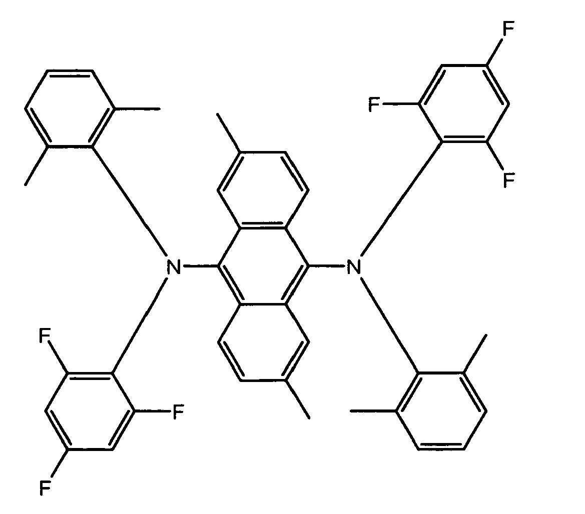

### Compound B4

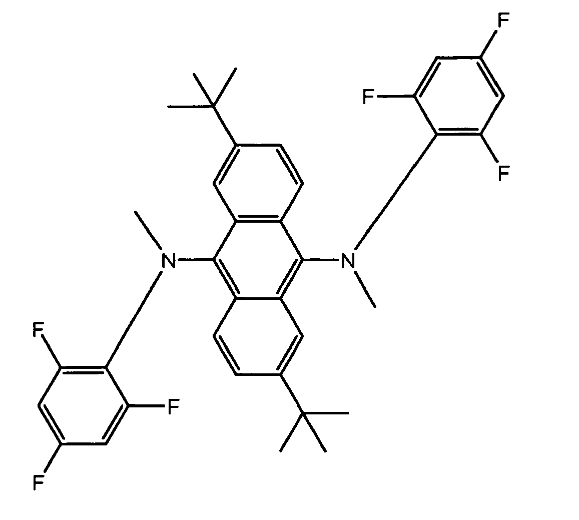

### Compound B5

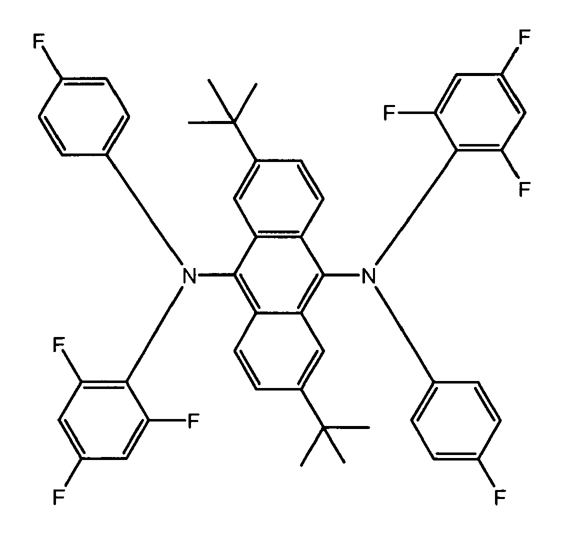

### Compound B6

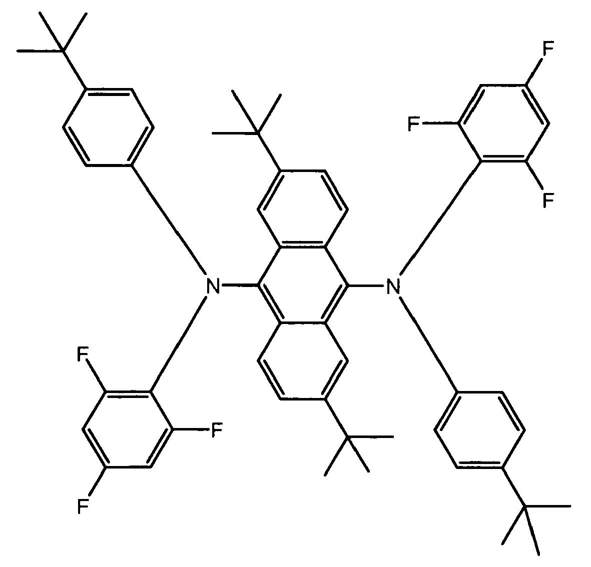

### Compound B7

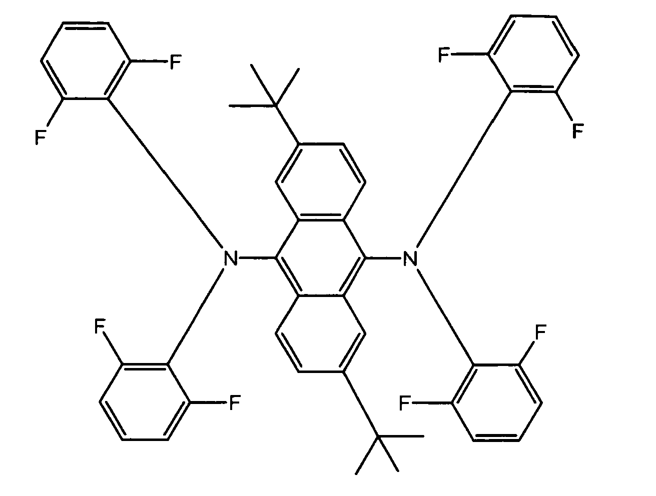

### Compound B8

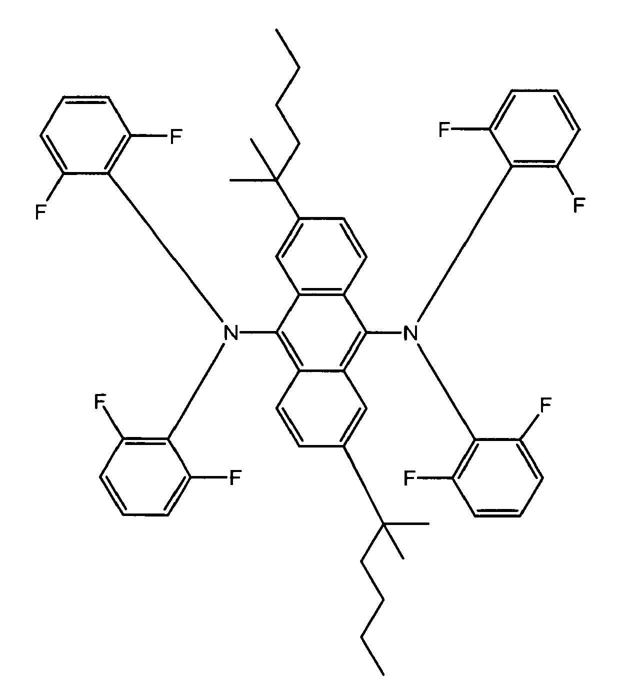

### 3. Synthesis

The blue luminescent materials described herein, are generally prepared according to the following scheme:

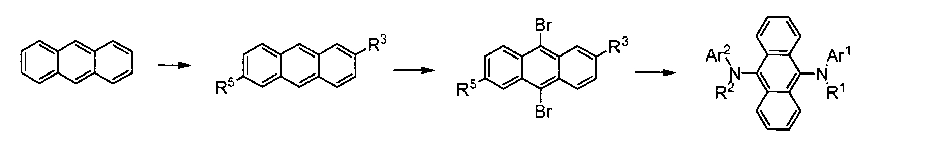

When R³ = R⁵, the first step involves alkylation of anthracene using Friedel Crafts chemistry with the appropriate alcohol, for example, t-butanol, 1-adamantanol or 1-methylcyclohexanol. This can be carried out in a solvent such as neat trifluoroacetic acid, generally with heating, followed by isolation and chromatographic purification. For some of the compounds, the substituted anthracene is commercially available, such as 2-t-butylanthracene.

The substituted anthracene can then be brominated, such as by using Br₂ in CCl₄. For compounds where R³ = R⁵ = H, the dibromide is commercially available.

The brominated product is then reacted with the appropriate amine with a Pd catalyst. The amine itself can also be prepared by Pd catalyzed amination.

In cases where the R³ = R⁵ = alkoxy group, the substituted anthracene intermediate can be prepared by etherification of 2,6-dihydroxyanthraquinone, followed by hydride reduction.

### 4. Devices

Organic electronic devices that may benefit from having one or more layers comprising the blue luminescent materials described herein include, but are not limited to, (1) devices that convert electrical energy into radiation (e.g., a light-emitting diode, light emitting diode display, or diode laser), (2) devices that detect signals through electronics processes (e.g., photodetectors, photoconductive cells, photoresistors, photoswitches, phototransistors, phototubes, IR detectors), (3) devices that convert radiation into electrical energy, (e.g., a photovoltaic device or solar cell), and (4) devices that include one or more electronic components that include one or more organic semi-conductor layers (e.g., a transistor or diode).

One illustration of an organic electronic device structure is shown in Figure 1. The device 100 has a first electrical contact layer, an anode layer 110 and a second electrical contact layer, a cathode layer 160, and a photoactive layer 140 between them. Adjacent to the anode is a buffer layer 120. Adjacent to the buffer layer is a hole transport layer 130, comprising hole transport material. Adjacent to the cathode may be an electron transport layer 150, comprising an electron transport material. As an option, devices may use one or more additional hole injection or hole transport layers (not shown) next to the anode 110 and/or one or more additional electron injection or electron transport layers (not shown) next to the cathode 160.

In one embodiment, the different layers have the following range of thicknesses: anode 110, 500-5000 Å, in one embodiment 1000-2000 Å; buffer layer 120, 50-2000 Å, in one embodiment 200-1000 Å; hole transport layer 120, 50-2000 Å, in one embodiment 200-1000 Å; photoactive layer 130, 10-2000 Å, in one embodiment 100-1000 Å; layer 140, 50-2000 Å, in one embodiment 100-1000 Å; cathode 150, 200-10000 Å, in one embodiment 300-5000 Å. The location of the electron-hole recombination zone in the device, and thus the emission spectrum of the device, can be affected by the relative thickness of each layer. The desired ratio of layer thicknesses will depend on the exact nature of the materials used.

### a. Photoactive Layer

The blue luminescent materials described herein are particularly suited as the photoactive material in the photoactive layer 140. They can be used alone, in combination with other luminescent materials, or in a host material.

In some embodiments, the host is a bis-condensed cyclic aromatic compound.

In some embodiments, the host is an anthracene derivative compound. In some embodiments the compound has the formula:

An - L - An

where:
An is an anthracene moiety;
L is a divalent connecting group.
In some embodiments of this formula, L is a single bond, -O-, -S-, - N(R)-, or an aromatic group. In some embodiments, An is a mono- or diphenylanthryl moiety.

In some embodiments, the host has the formula:

A - An - A

where:
An is an anthracene moiety;
A is an aromatic group.

In some embodiments, the host is a diarylanthracene. In some embodiments the compound is symmetrical and in some embodiments the compound is non-symmetrical.

In some embodiments, the host has the formula: 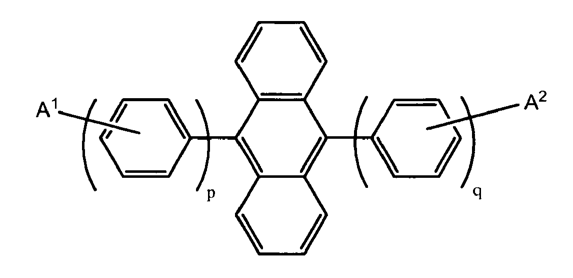 where:
A¹ and A² are the same or different at each occurrence and are selected from the group consisting of H, an aromatic group, and an alkenyl group, or A may represent one or more fused aromatic rings;
p and q are the same or different and are an integer from 1-3.
In some embodiments, the anthracene derivative is non-symmetrical. In some embodiments, p =2 and q = 1. In some embodiments, at least one of A¹ and A² is a naphthyl group.

In some embodiments, the host is selected from the group consisting of
H1 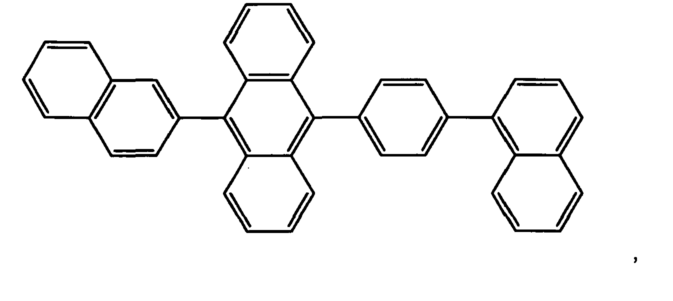 H2 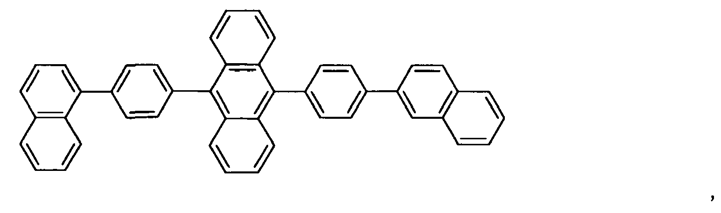 and combinations thereof.

### b. Other Device Layers

The other layers in the device can be made of any materials which are known to be useful in such layers.

The anode 110 is an electrode that is particularly efficient for injecting positive charge carriers. It can be made of, for example materials containing a metal, mixed metal, alloy, metal oxide or mixed-metal oxide, or it can be a conducting polymer, and mixtures thereof. Suitable metals include the Group 11 metals, the metals in Groups 4, 5, and 6, and the Group 8-10 transition metals. If the anode is to be light-transmitting, mixed-metal oxides of Groups 12, 13 and 14 metals, such as indium-tin-oxide, are generally used. The anode may also comprise an organic material such as polyaniline as described in "Flexible light-emitting diodes made from soluble conducting polymer," Nature vol. 357, pp 477 479 (11 June 1992). At least one of the anode and cathode should be at least partially transparent to allow the generated light to be observed.

The buffer layer 120 comprises buffer material and may have one or more functions in an organic electronic device, including but not limited to, planarization of the underlying layer, charge transport and/or charge injection properties, scavenging of impurities such as oxygen or metal ions, and other aspects to facilitate or to improve the performance of the organic electronic deviceThe buffer layer can be formed with polymeric materials, such as polyaniline (PANI) or polyethylenedioxythiophene (PEDOT), which are often doped with protonic acids. The protonic acids can be, for example, poly(styrenesulfonic acid), poly(2-acrylamido-2-methyl-1-propanesulfonic acid), and the like.

The buffer layer can comprise charge transfer compounds, and the like, such as copper phthalocyanine and the tetrathiafulvalene-tetracyanoquinodimethane system (TTF-TCNQ).

In some embodiments, the buffer layer comprises at least one electrically conductive polymer and at least one fluorinated acid polymer.

In some embodiments, the electrically conductive polymer will form a film which has a conductivity of at least 10⁻⁷ S/cm. The monomer from which the conductive polymer is formed, is referred to as a "precursor monomer". A copolymer will have more than one precursor monomer. In some embodiments, the conductive polymer is made from at least one precursor monomer selected from thiophenes, selenophenes, tellurophenes, pyrroles, anilines, and polycyclic aromatics. The polymers made from these monomers are referred to herein as polythiophenes, poly(selenophenes), poly(tellurophenes), polypyrroles, polyanilines, and polycyclic aromatic polymers, respectively. The term "polycyclic aromatic" refers to compounds having more than one aromatic ring. The rings may be joined by one or more bonds, or they may be fused together. The term "aromatic ring" is intended to include heteroaromatic rings. A "polycyclic heteroaromatic" compound has at least one heteroaromatic ring. In some embodiments, the polycyclic aromatic polymers are poly(thienothiophenes).

The fluorinated acid polymer can be any polymer which is fluorinated and has acidic groups with acidic protons. The term includes partially and fully fluorinated materials. In some embodiments, the fluorinated acid polymer is highly fluorinated. The term "highly fluorinated" means that at least 50% of the available hydrogens bonded to a carbon, have been replaced with fluorine. The acidic groups supply an ionizable proton. In some embodiments, the acidic proton has a pKa of less than 3. In some embodiments, the acidic proton has a pKa of less than 0. In some embodiments, the acidic proton has a pKa of less than -5. The acidic group can be attached directly to the polymer backbone, or it can be attached to side chains on the polymer backbone. Examples of acidic groups include, but are not limited to, carboxylic acid groups, sulfonic acid groups, sulfonimide groups, phosphoric acid groups, phosphonic acid groups, and combinations thereof. The acidic groups can all be the same, or the polymer may have more than one type of acidic group.

In some embodiments the fluorinated acid polymer is water-soluble. In some embodiments, the fluorinated acid polymer is dispersible in water. In some embodiments, the fluorinated acid polymer is organic solvent wettable.

In some embodiments, fluorinated acid polymer has a polymer backbone which is fluorinated. Examples of suitable polymeric backbones include, but are not limited to, polyolefins, polyacrylates, polymethacrylates, polyimides, polyamides, polyaramids, polyacrylamides, polystyrenes, and copolymers thereof. In some embodiments, the polymer backbone is highly fluorinated. In some embodiments, the polymer backbone is fully fluorinated.

In some embodiments, the acidic groups are sulfonic acid groups or sulfonimide groups. A sulfonimide group has the formula:

-SO₂-NH-SO₂-R

where R is an alkyl group.

In some embodiments, the acidic groups are on a fluorinated side chain. In some embodiments, the fluorinated side chains are selected from alkyl groups, alkoxy groups, amido groups, ether groups, and combinations thereof.

In some embodiments, the fluorinated acid polymer has a fluorinated olefin backbone, with pendant fluorinated ether sulfonate, fluorinated ester sulfonate, or fluorinated ether sulfonimide groups. In some embodiments, the polymer is a copolymer of 1,1-difluoroethylene and 2-(1,1-difluoro-2-(trifluoromethyl)allyloxy)-1,1,2,2-tetrafluoroethanesulfonic acid. In some embodiments, the polymer is a copolymer of ethylene and 2-(2-(1,2,2-trifluorovinyloxy)-1,1,2,3,3,3-hexafluoropropoxy)-1,1,2,2-tetrafluoroethanesulfonic acid. These copolymers can be made as the corresponding sulfonyl fluoride polymer and then can be converted to the sulfonic acid form.

In some embodiments, the fluorinated acid polymer is homopolymer or copolymer of a fluorinated and partially sulfonated poly(arylene ether sulfone). The copolymer can be a block copolymer. Examples of comonomers include, but are not limited to butadiene, butylene, isobutylene, styrene, and combinations thereof.

In some embodiments, the buffer layer is made from an aqueous dispersion of an electrically conducting polymer and a colloid-forming polymeric acid. Such materials have been described in, for example, published U.S. patent applications 2004-0102577, 2004-0127637, and 2005-0205860.

The hole transport layer 130 is a layer which facilitates migration of positive charges through the thickness of the layer with relative efficiency and small loss of charge. Examples of hole transport materials for the hole transport layer have been summarized for example, in Kirk-Othmer Encyclopedia of Chemical Technology, Fourth Edition, Vol. 18, p. 837-860, 1996, by Y. Wang. Both hole transporting small molecules and polymers can be used. Commonly used hole transporting molecules include, but are not limited to: 4,4',4"-tris(N,N-diphenyl-amino)-triphenylamine (TDATA); 4,4',4"-tris(N-3-methylphenyl-N-phenyl-amino)-triphenylamine (MTDATA); N,N'-diphenyl-N,N'-bis(3-methylphenyl)-[1,1'-biphenyl]-4,4'-diamine (TPD); 4, 4'-bis(carbazol-9-yl)biphenyl (CBP); 1,3-bis(carbazol-9-yl)benzene (mCP); 1,1-bis[(di-4-tolylamino) phenyl]cyclohexane (TAPC); N,N'-bis(4-methylphenyl)-N,N'-bis(4-ethylphenyl)-[1,1'-(3,3'-dimethyl)biphenyl]-4,4'-diamine (ETPD); tetrakis-(3-methylphenyl)-N,N,N',N'-2,5-phenylenediamine (PDA); α-phenyl-4-N,N-diphenylaminostyrene (TPS); p-(diethylamino)benzaldehyde diphenylhydrazone (DEH); triphenylamine (TPA); bis[4-(N,N-diethylamino)-2-methylphenyl](4-methylphenyl)methane (MPMP); 1-phenyl-3-[p-(diethylamino)styryl]-5-[p-(diethylamino)phenyl] pyrazoline (PPR or DEASP); 1,2-trans-bis(9H-carbazol-9-yl)cyclobutane (DCZB); N,N,N',N'-tetrakis(4-methylphenyl)-(1,1'-biphenyl)-4,4'-diamine (TTB); N,N'-bis(naphthalen-1-yl)-N,N'-bis-(phenyl)benzidine (α-NPB); and porphyrinic compounds, such as copper phthalocyanine. Commonly used hole transporting polymers include, but are not limited to, polyvinylcarbazole, (phenylmethyl)polysilane, poly(dioxythiophenes), polyanilines, and polypyrroles. It is also possible to obtain hole transporting polymers by doping hole transporting molecules such as those mentioned above into polymers such as polystyrene and polycarbonate.

In some embodiments, the hole transport layer comprises a hole transport polymer. In some embodiments, the hole transport polymer is a distyrylaryl compound. In some embodiments, the aryl group has two or more fused aromatic rings. In some embodiments, the aryl group is an acene. The term "acene" as used herein refers to a hydrocarbon parent component that contains two or more *ortho*-fused benzene rings in a straight linear arrangement.

In some embodiments, the hole transport polymer is an arylamine polymer. In some embodiments, it is a copolymer of fluorene and arylamine monomers.

In some embodiments, the polymer has crosslinkable groups. In some embodiments, crosslinking can be accomplished by a heat treatment and/or exposure to UV or visible radiation. Examples of crosslinkable groups include, but are not limited to vinyl, acrylate, perfluorovinylether, 1-benzo-3,4-cyclobutane, siloxane, and methyl esters. Crosslinkable polymers can have advantages in the fabrication of solution-process OLEDs. The application of a soluble polymeric material to form a layer which can be converted into an insoluble film subsequent to deposition, can allow for the fabrication of multilayer solution-processed OLED devices free of layer dissolution problems.

Examples of crosslinkable polymers can be found in, for example, published US patent application 2005-0184287 and published PCT application WO 2005/052027.

In some embodiments, the hole transport layer comprises a polymer which is a copolymer of 9,9-dialkylfluorene and triphenylamine. In some embodiments, the polymer is a copolymer of 9,9-dialkylfluorene and 4,4'-bis(diphenylamino)biphenyl. In some embodiments, the polymer is a copolymer of 9,9-dialkylfluorene and TPB. In some embodiments, the polymer is a copolymer of 9,9-dialkylfluorene and NPB. In some embodiments, the copolymer is made from a third comonomer selected from (vinylphenyl)diphenylamine and 9,9-distyrylfluorene or 9,9-di(vinylbenzyl)fluorene.

The electron transport layer 150 is a layer which facilitates migration of negative charges through the thickness of the layer with relative efficiency and small loss of charge. Examples of electron transport materials which can be used in the optional electron transport layer 140, include metal chelated oxinoid compounds, such as tris(8-hydroxyquinolato)aluminum (AlQ), bis(2-methyl-8-quinolinolato)(p-phenylphenolato) aluminum (BAlq), tetrakis-(8-hydroxyquinolato)hafnium (HfQ) and tetrakis-(8-hydroxyquinolato)zirconium (ZrQ); and azole compounds such as 2- (4-biphenylyl)-5-(4-t-butylphenyl)-1,3,4-oxadiazole (PBD), 3-(4-biphenylyl)-4-phenyl-5-(4-t-butylphenyl)-1,2,4-triazole (TAZ), and 1,3,5-tri(phenyl-2-benzimidazole)benzene (TPBI); quinoxaline derivatives such as 2,3-bis(4-fluorophenyl)quinoxaline; phenanthrolines such as 4,7-diphenyl-1,10-phenanthroline (DPA) and 2,9-dimethyl-4,7-diphenyl-1,10-phenanthroline (DDPA); and mixtures thereof.

The cathode 160, is an electrode that is particularly efficient for injecting electrons or negative charge carriers. The cathode can be any metal or nonmetal having a lower work function than the anode. Materials for the cathode can be selected from alkali metals of Group 1 (e.g., Li, Cs), the Group 2 (alkaline earth) metals, the Group 12 metals, including the rare earth elements and lanthanides, and the actinides. Materials such as aluminum, indium, calcium, barium, samarium and magnesium, as well as combinations, can be used.

Alkali metal-containing inorganic compounds, such as LiF, CsF, Cs₂O and Li₂O can also be deposited between the organic layer 150 and the cathode layer 160 to lower the operating voltage. This layer, not shown, may be referred to as an electron injection layer.

### c. Device Fabrication

The device layers can be formed by any deposition technique, or combinations of techniques, including vapor deposition, liquid deposition, and thermal transfer.

In some embodiments, the device is fabricated by liquid deposition of the buffer layer, the hole transport layer, and the photoactive layer, and by vapor deposition of the anode, the electron transport layer, an electron injection layer and the cathode.

The buffer layer can be deposited from any liquid medium in which it is dissolved or dispersed and from which it will form a film. In one embodiment, the liquid medium consists essentially of one or more organic solvents. In one embodiment, the liquid medium consists essentially of water or water and an organic solvent. In one embodiment the organic solvent is selected from the group consisting of alcohols, ketones, cyclic ethers, and polyols. In one embodiment, the organic liquid is selected from dimethylacetamide ("DMAc"), N-methylpyrrolidone ("NMP"), dimethylformamide ("DMF"), ethylene glycol ("EG"), aliphatic alcohols, and mixtures thereof. The buffer material can be present in the liquid medium in an amount from 0.5 to 10 percent by weight. Other weight percentages of buffer material may be used depending upon the liquid medium. The buffer layer can be applied by any continuous or discontinuous liquid deposition technique. In one embodiment, the buffer layer is applied by spin coating. In one embodiment, the buffer layer is applied by ink jet printing. After liquid deposition, the liquid medium can be removed in air, in an inert atmosphere, or by vacuum, at room temperature or with heating. In one embodiment, the layer is heated to a temperature less than 275°C. In one embodiment, the heating temperature is between 100°C and 275°C. In one embodiment, the heating temperature is between 100°C and 120°C. In one embodiment, the heating temperature is between 120°C and 140°C. In one embodiment, the heating temperature is between 140°C and 160°C.In one embodiment, the heating temperature is between 160°C and 180°C. In one embodiment, the heating temperature is between 180°C and 200°C. In one embodiment, the heating temperature is between 200°C and 220°C.ln one embodiment, the heating temperature is between 190°C and 220°C. In one embodiment, the heating temperature is between 220°C and 240°C. In one embodiment, the heating temperature is between 240°C and 260°C. In one embodiment, the heating temperature is between 260°C and 275°C. The heating time is dependent upon the temperature, and is generally between 5 and 60 minutes. In one embodiment, the final layer thickness is between 5 and 200 nm. In one embodiment, the final layer thickness is between 5 and 40 nm. In one embodiment, the final layer thickness is between 40 and 80 nm. In one embodiment, the final layer thickness is between 80 and 120 nm. In one embodiment, the final layer thickness is between 120 and 160 nm. In one embodiment, the final layer thickness is between 160 and 200 nm.

The hole transport layer can be deposited from any liquid medium in which it is dissolved or dispersed and from which it will form a film. In one embodiment, the liquid medium consists essentially of one or more organic solvents. In one embodiment, the liquid medium consists essentially of water or water and an organic solvent. In one embodiment the organic solvent is an aromatic solvent. In one embodiment, the organic liquid is selected from chloroform, dichloromethane, chlorobenzene, dichlorobenzene, toluene, xylene, mesitylene, anisole, and mixtures thereof. The hole transport material can be present in the liquid medium in a concentration of 0.2 to 2 percent by weight. Other weight percentages of hole transport material may be used depending upon the liquid medium. The hole transport layer can be applied by any continuous or discontinuous liquid deposition technique. In one embodiment, the hole transport layer is applied by spin coating. In one embodiment, the hole transport layer is applied by ink jet printing. After liquid deposition, the liquid medium can be removed in air, in an inert atmosphere, or by vacuum, at room temperature or with heating. In one embodiment, the layer is heated to a temperature of 300°C or less. In one embodiment, the heating temperature is between 170°C and 275°C. In one embodiment, the heating temperature is between 170°C and 200°C. In one embodiment, the heating temperature is between 190°C and 220°C. In one embodiment, the heating temperature is between 210°C and 240°C. In one embodiment, the heating temperature is between 230°C and 270°C. In one embodiment, the heating temperature is between 270°C and 300°C. The heating time is dependent upon the temperature, and is generally between 5 and 60 minutes. In one embodiment, the final layer thickness is between 5 and 50 nm. In one embodiment, the final layer thickness is between 5 and 15 nm. In one embodiment, the final layer thickness is between 15 and 25 nm. In one embodiment, the final layer thickness is between 25 and 35 nm. In one embodiment, the final layer thickness is between 35 and 50 nm.

The photoactive layer can be deposited from any liquid medium in which it is dissolved or dispersed and from which it will form a film. In one embodiment, the liquid medium consists essentially of one or more organic solvents. In one embodiment, the liquid medium consists essentially of water or water and an organic solvent. In one embodiment the organic solvent is an aromatic solvent. In one embodiment, the organic solvent is selected from chloroform, dichloromethane, toluene, anisole, 2-butanone, 3-pentanone, butyl acetate, acetone, xylene, mesitylene, chlorobenzene, tetrahydrofuran, diethyl ether, trifluorotoluene, and mixtures thereof. The photoactive material can be present in the liquid medium in a concentration of 0.2 to 2 percent by weight. Other weight percentages of photoactive material may be used depending upon the liquid medium. The photoactive layer can be applied by any continuous or discontinuous liquid deposition technique. In one embodiment, the photoactive layer is applied by spin coating. In one embodiment, the photoactive layer is applied by ink jet printing. After liquid deposition, the liquid medium can be removed in air, in an inert atmosphere, or by vacuum, at room temperature or with heating. Optimal baking conditions depend on the vapor pressure properties of the liquids being removed and their molecular interaction with the liquids. In one embodiment, the deposited layer is heated to a temperature that is greater than the Tg of the material having the highest Tg. In one embodiment, the deposited layer is heated between 10 and 20 °C higher than the Tg of the material having the highest Tg. In one embodiment, the deposited layer is heated to a temperature that is less than the Tg of the material having the lowest Tg. In one embodiment, the heating temperature is at least 10°C less than the lowest Tg. In one embodiment, the heating temperature is at least 20°C less than the lowest Tg. In one embodiment, the heating temperature is at least 30°C less than the lowest Tg. In one embodiment, the heating temperature is between 50°C and 150°C. In one embodiment, the heating temperature is between 50°C and 75°C. In one embodiment, the heating temperature is between 75°C and 100°C. In one embodiment, the heating temperature is between 100°C and 125°C. In one embodiment, the heating temperature is between 125°C and 150°C. The heating time is dependent upon the temperature, and is generally between 5 and 60 minutes. In one embodiment, the final layer thickness is between 25 and 100 nm. In one embodiment, the final layer thickness is between 25 and 40 nm. In one embodiment, the final layer thickness is between 40 and 65 nm. In one embodiment, the final layer thickness is between 65 and 80 nm. In one embodiment, the final layer thickness is between 80 and 100 nm.

The electron transport layer can be deposited by any vapor deposition method. In one embodiment, it is deposited by thermal evaporation under vacuum. In one embodiment, the final layer thickness is between 1 and 100 nm. In one embodiment, the final layer thickness is between 1 and 15 nm. In one embodiment, the final layer thickness is between 15 and 30 nm. In one embodiment, the final layer thickness is between 30 and 45 nm. In one embodiment, the final layer thickness is between 45 and 60 nm. In one embodiment, the final layer thickness is between 60 and 75 nm. In one embodiment, the final layer thickness is between 75 and 90 nm. In one embodiment, the final layer thickness is between 90 and 100 nm.

The electron injection layer can be deposited by any vapor deposition method. In one embodiment, it is deposited by thermal evaporation under vacuum. In one embodiment, the vacuum is less than 10⁻⁶ torr. In one embodiment, the vacuum is less than 10⁻⁷ torr. In one embodiment, the vacuum is less than 10⁻⁸ torr. In one embodiment, the material is heated to a temperature in the range of 100°C to 400°C; 150°C to 350°C preferably. The vapor deposition rates given herein are in units of Angstroms per second. In one embodiment, the material is deposited at a rate of 0.5 to 10 Ǻ/sec. In one embodiment, the material is deposited at a rate of 0.5 to 1 Ǻ/sec. In one embodiment, the material is deposited at a rate of 1 to 2 Ǻ/sec. In one embodiment, the material is deposited at a rate of 2 to 3 Ǻ/sec. In one embodiment, the material is deposited at a rate of 3 to 4 Ǻ/sec. In one embodiment, the material is deposited at a rate of 4 to 5 Ǻ/sec. In one embodiment, the material is deposited at a rate of 5 to 6 Ǻ/sec. In one embodiment, the material is deposited at a rate of 6 to 7 Ǻ/sec. In one embodiment, the material is deposited at a rate of 7 to 8 Ǻ/sec. In one embodiment, the material is deposited at a rate of 8 to 9 Ǻ/sec. In one embodiment, the material is deposited at a rate of 9 to 10 Ǻ/sec. In one embodiment, the final layer thickness is between 0.1 and 3 nm. In one embodiment, the final layer thickness is between 0.1 and 1 nm. In one embodiment, the final layer thickness is between 1 and 2 nm. In one embodiment, the final layer thickness is between 2 and 3 nm.

The cathode can be deposited by any vapor deposition method. In one embodiment, it is deposited by thermal evaporation under vacuum. In one embodiment, the vacuum is less than 10⁻⁶ torr. In one embodiment, the vacuum is less than 10⁻⁷ torr. In one embodiment, the vacuum is less than 10⁻⁸ torr. In one embodiment, the material is heated to a temperature in the range of 100°C to 400°C; 150°C to 350°C preferably. In one embodiment, the material is deposited at a rate of 0.5 to 10 Å/sec. In one embodiment, the material is deposited at a rate of 0.5 to 1 Å/sec. In one embodiment, the material is deposited at a rate of 1 to 2 Ǻ/sec. In one embodiment, the material is deposited at a rate of 2 to 3 Ǻ/sec. In one embodiment, the material is deposited at a rate of 3 to 4 Ǻ/sec. In one embodiment, the material is deposited at a rate of 4 to 5 Ǻ/sec. In one embodiment, the material is deposited at a rate of 5 to 6 Ǻ/sec. In one embodiment, the material is deposited at a rate of 6 to 7 Ǻ/sec. In one embodiment, the material is deposited at a rate of 7 to 8 Ǻ/sec. In one embodiment, the material is deposited at a rate of 8 to 9 Ǻ/sec. In one embodiment, the material is deposited at a rate of 9 to 10 Ǻ/sec. In one embodiment, the final layer thickness is between 10 and 10000 nm. In one embodiment, the final layer thickness is between 10 and 1000 nm. In one embodiment, the final layer thickness is between 10 and 50 nm. In one embodiment, the final layer thickness is between 50 and 100 nm. In one embodiment, the final layer thickness is between 100 and 200 nm. In one embodiment, the final layer thickness is between 200 and 300 nm. In one embodiment, the final layer thickness is between 300 and 400 nm. In one embodiment, the final layer thickness is between 400 and 500 nm. In one embodiment, the final layer thickness is between 500 and 600 nm. In one embodiment, the final layer thickness is between 600 and 700 nm. In one embodiment, the final layer thickness is between 700 and 800 nm. In one embodiment, the final layer thickness is between 800 and 900 nm. In one embodiment, the final layer thickness is between 900 and 1000 nm. In one embodiment, the final layer thickness is between 1000 and 2000 nm. In one embodiment, the final layer thickness is between 2000 and 3000 nm. In one embodiment, the final layer thickness is between 3000 and 4000 nm. In one embodiment, the final layer thickness is between 4000 and 5000 nm. In one embodiment, the final layer thickness is between 5000 and 6000 nm. In one embodiment, the final layer thickness is between 6000 and 7000 nm. In one embodiment, the final layer thickness is between 7000 and 8000 nm. In one embodiment, the final layer thickness is between 8000 and 9000 nm. In one embodiment, the final layer thickness is between 9000 and 10000 nm.

### EXAMPLES

The concepts described herein will be further described in the following examples, which do not limit the scope of the invention described in the claims.

### Example 1.

This example illustrates the preparation of Compound B7.

### a) Preparation of 2, 2', 6', 6'-tetrafluorodiphenylamine

3.86g of the 2,6-difluoro-bromobenzene(20mM) in a nitrogen filled glove box was added to 2.6g (20mM) 2,6-difluoroaniline and all stirred into 50mL dry toluene. 0.21g Pd₂DBA₃ (0.21 mM), 0.09g P(t-Bu)₃ (0.42mM) and 2.0g t-BuONa (21 mM) were added. Upon addition of these catalyst materials, there is a sharp exotherm. The dark yellow brown mixture was then heated in the glove box at 80°C for 4 hrs.

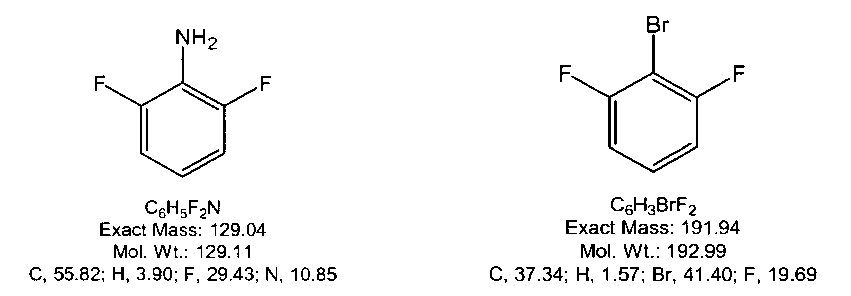

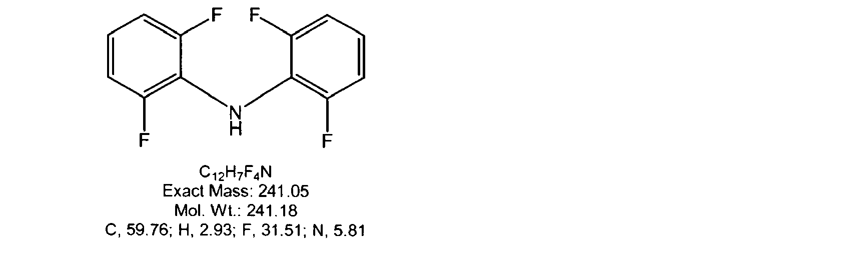

The reaction mixture was then cooled and removed from the glove box to work up by silica chromatography using methylene chloride eluent to generate the desired secondary amine as a dark green/brown oil with structure confirmed by nmr. Yield ∼60%.

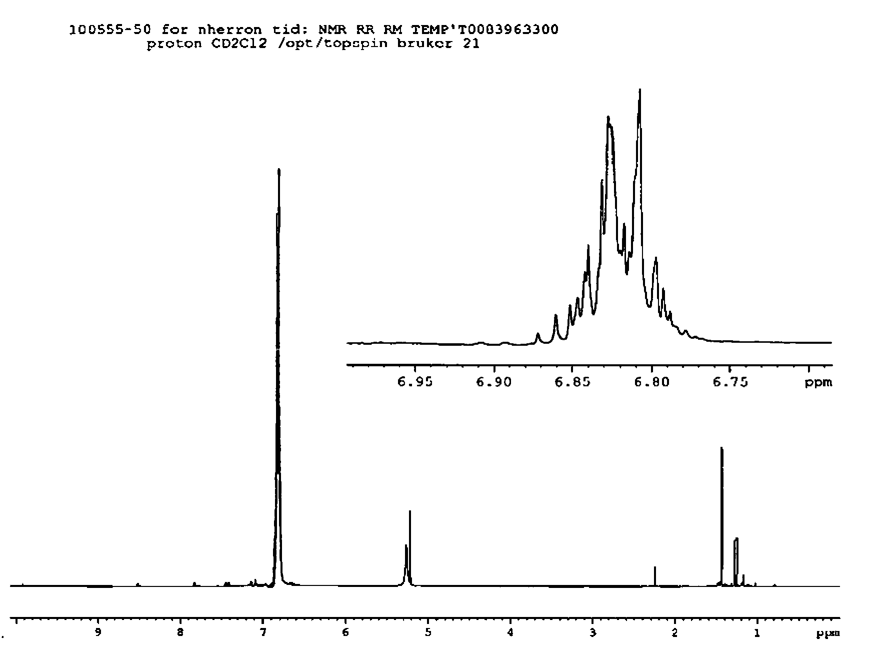

### b) Amination of di-t-butyl-anthracene to generate B7

In a nitrogen filled glove box, 1.0g of 2,6-di-t-butyl-9,10-dibromoanthracene (2.2mM) and 1.06g (4.4mM) sec amine from above (a) were mixed in 25mL dry toluene. 0.2g Pd₂DBA₃ (0.2mM), 0.08g P(t-Bu)₃ (0.4mM) and 0.5g t-BuONa (5mM) were all added to the stirred toluene reaction mixture. The reaction mixture was then heated in the glove box at 80°C for 1 hr. The solution immediately is dark purple but on reaching ∼80°C it is yellow/red/brown and noticeably thicker. After 90mins, the reaction was cooled and worked up by removing from the glove box and filtering through neutral alumina to removed brown and green colored impurities. The eluent (methylene chloride) is pale yellow and is brightly blue photoluminescent. Addition of methanol to the concentrated solution results in a pale yellow crystalline precipitate which is blue luminescent. Yield is ∼1.0g. Solution in toluene is very pale yellow and blue luminescent in room light. 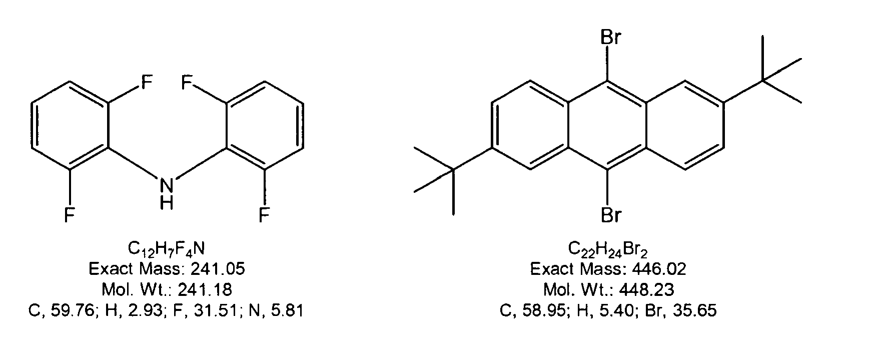 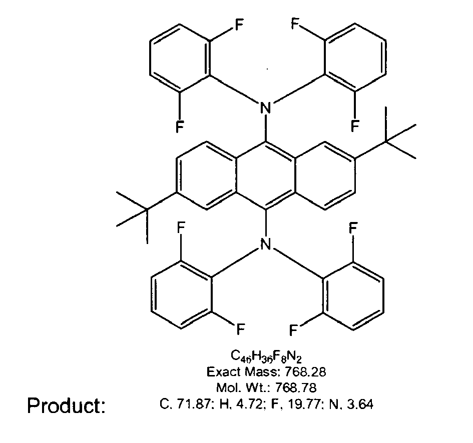 The recovered material was chromatographed through a column of FLORISIL eluting with methylene chloride to clean up further then submit for nmr which confirms the expected product

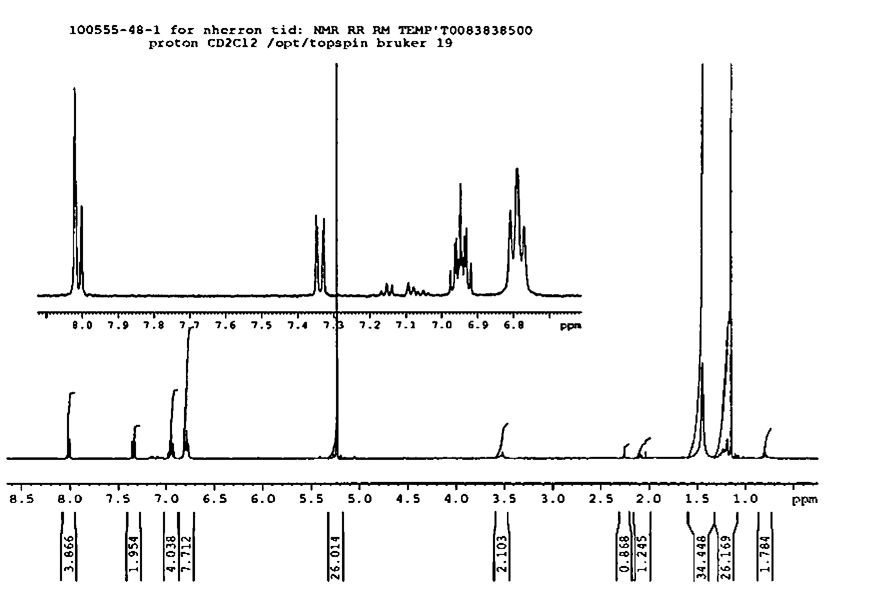

Note that not all of the activities described above in the general description or the examples are required, that a portion of a specific activity may not be required, and that one or more further activities may be performed in addition to those described. Still further, the order in which activities are listed are not necessarily the order in which they are performed.

In the foregoing specification, the concepts have been described with reference to specific embodiments. However, one of ordinary skill in the art appreciates that various modifications and changes can be made without departing from the scope of the invention as set forth in the claims below. Accordingly, the specification and figures are to be regarded in an illustrative rather than a restrictive sense, and all such modifications are intended to be included within the scope of invention.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.
It is to be appreciated that certain features are, for clarity, described herein in the context of separate embodiments, may also be provided in combination in a single embodiment. Conversely, various features that are, for brevity, described in the context of a single embodiment, may also be provided separately or in any subcombination. Further, reference to values stated in ranges include each and every value within that range.

## Claims

1. A blue luminescent material having Formula I 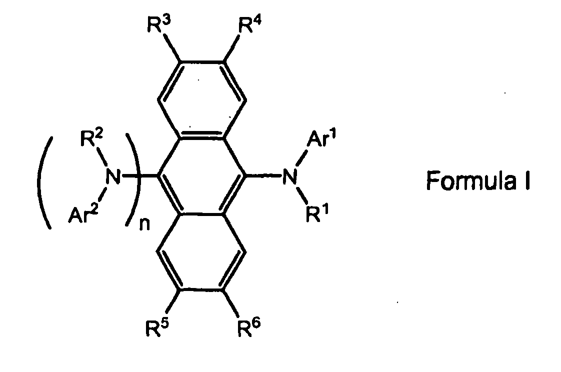 wherein:
Ar1 and Ar2 are the same or different and each is an aryl group having at least two electron-withdrawing substituents selected from the group consisting of fluoro, cyano, perfluoroalkyl, nitro, -SO₂R, and combinations thereof, where R is alkyl or perfluoroalkyl;
R¹ and R² are the same or different and are selected from the group consisting of alkyl, and aryl;
R³, R⁴, R⁵ and R⁶ are the same or different and are selected from the group consisting of hydrogen, alkyl, and alkoxy; and
n is 1.

2. The luminescent material of Claim 1, wherein Ar1 and Ar2 are selected from the group consisting of phenyl, biphenyl, naphthyl, and binaphthyl.

3. The luminescent material of Claim 1, wherein at least one of R¹ and R² is an alkyl.

4. The luminescent material of Claim 1, wherein both R¹ and R² are aryl groups selected from the group consisting of phenyl, biphenyl, naphthyl, and binaphthyl.

5. The luminescent material of Claim 4, wherein the aryl group has at least one substituent selected from the group consisting of an electron-withdrawing group, alkyl, and alkoxy, wherein the electron-withdrawing group is selected from the group consisting of fluoro, cyano, perfluoroalkyl, nitro, -SO₂R, and combinations thereof, where R is alkyl or perfluoroalkyl;

6. The luminescent material of Claim 1, wherein R³ = R⁵ = H, and R⁴ = R⁶ and is selected from the group consisting of branched alkyl and cycloalkyl.

7. The luminescent material of Claim 1, wherein R⁴ = R⁵ = H, and R³ = R⁶ and is selected from the group consisting of branched alkyl and cycloalkyl.

8. A blue luminescent material selected from the group consisting of
Compound B1 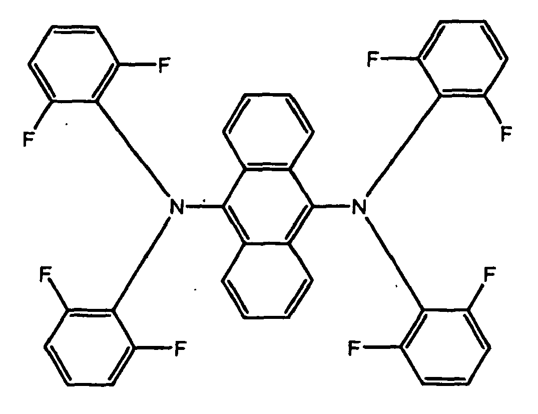 Compound B2 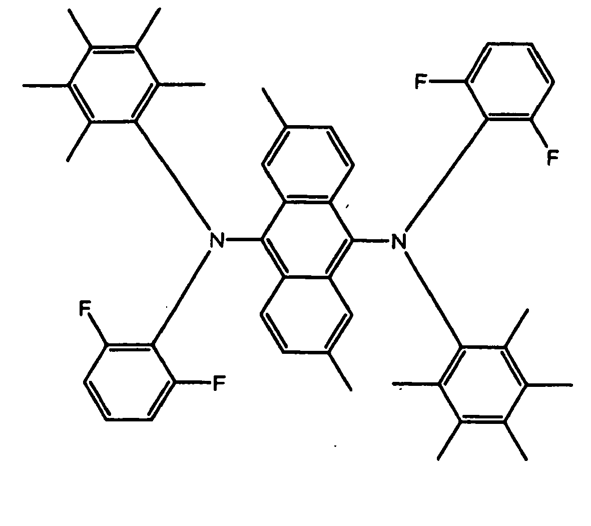 Compound B3 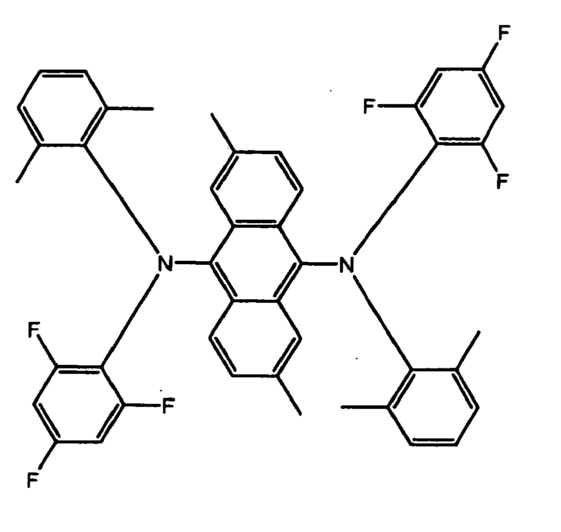 Compound B4 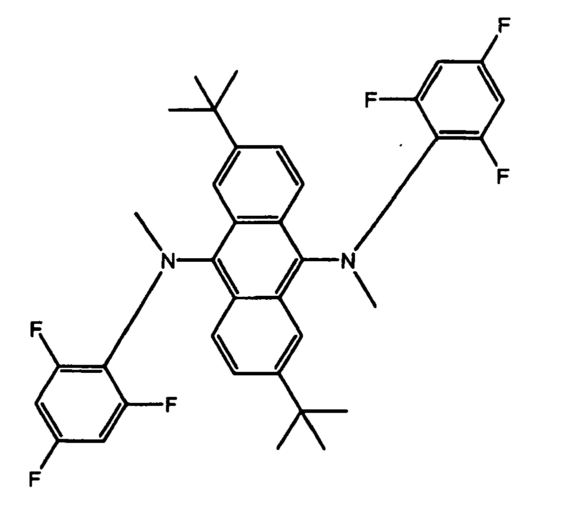 Compound B5 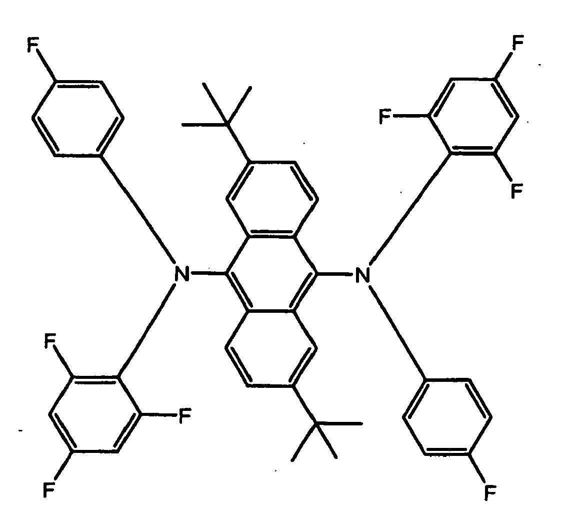 Compound B6 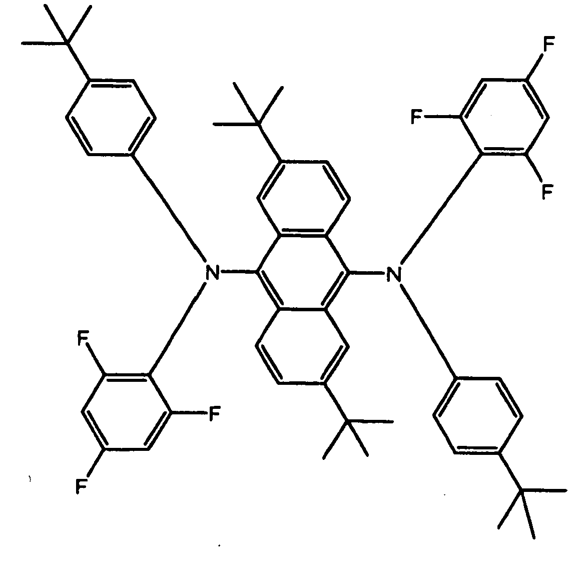 Compound B7 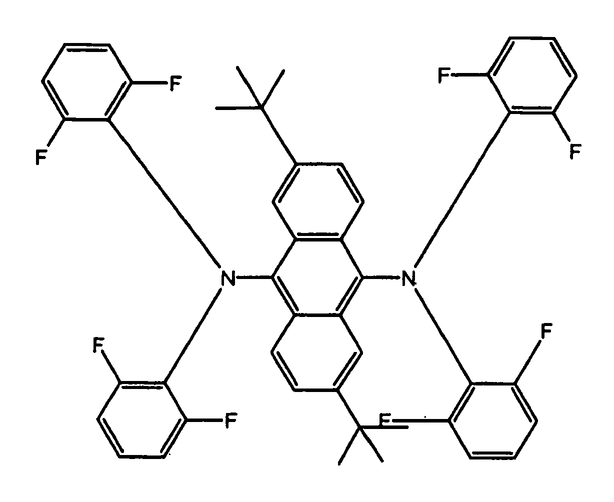 Compound B8

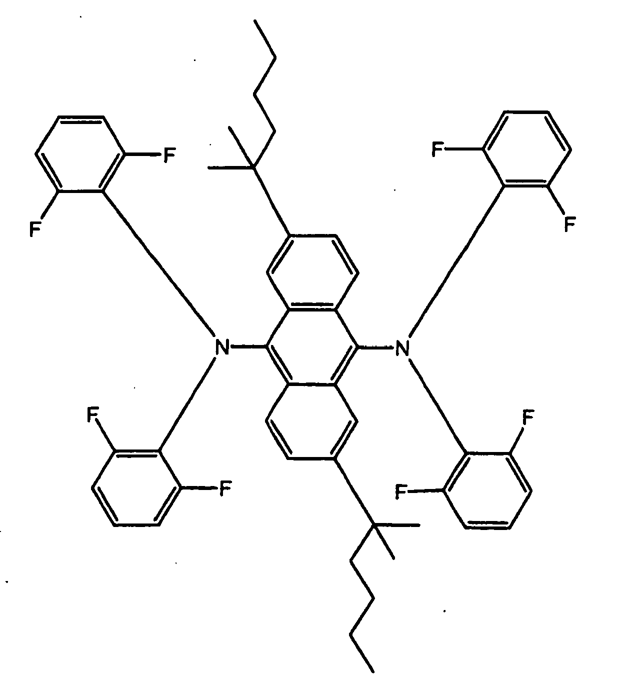

9. An organic electronic device comprising a first electrical contact, a second electrical contact and a photoactive layer therebetween, the photoactive layer comprising a blue luminescent material having Formula I 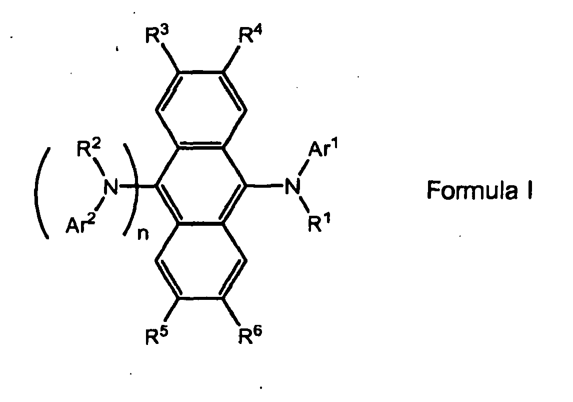 wherein:
Ar1 and Ar2 are the same or different and each is an aryl group having at least two electron-withdrawing substituents selected from the group consisting of fluoro, cyano, perfluoroalkyl, nitro, -SO₂R, and combinations thereof, where R is alkyl or perfluoroalkyl;
R¹ and R² are the same or different and are selected from the group consisting of alkyl, and aryl;
R³, R⁴, R⁵ and R⁶ are the same or different and are selected from the group consisting of hydrogen, alkyl, and alkoxy; and
n is 1.

## Patentansprüche

1. Blauleuchtendes Material, das die Formel I 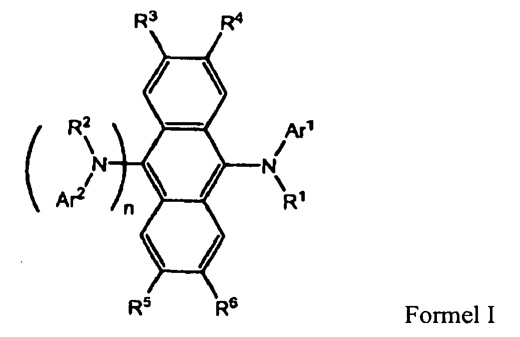 ausweist, wobei:
Ar1 und Ar2 gleich oder verschieden sind und jedes eine Arylgruppe ist, die mindestens zwei elektronenziehende Substituenten aufweist ausgewählt aus der Gruppe bestehend aus Fluor, Cyan, Perfluoralkyl, Nitro, -SO₂R und Kombinationen davon, wobei R Alkyl oder Perfluoralkyl ist;
R¹ und R² gleich oder verschieden und aus der Gruppe ausgewählt sind bestehend aus Alkyl und Aryl;
R³, R⁴, R⁵ und R⁶ gleich oder verschieden und aus der Gruppe ausgewählt sind bestehend aus Wasserstoff, Alkyl und Alkoxy; und
n 1 beträgt.

2. Leuchtendes Material nach Anspruch 1, wobei Ar1 und Ar2 aus der Gruppe ausgewählt sind bestehend aus Phenyl, Biphenyl, Naphthyl und Binaphthyl.

3. Leuchtendes Material nach Anspruch 1, wobei mindestens eines von R¹ und R² ein Alkyl ist.

4. Leuchtendes Material nach Anspruch 1, wobei sowohl R¹ als auch R² Arylgruppen sind ausgewählt aus der Gruppe bestehend aus Phenyl, Biphenyl, Naphthyl und Binaphthyl.

5. Leuchtendes Material nach Anspruch 4, wobei die Arylgruppe mindestens einen Substituenten aufweist ausgewählt aus der Gruppe bestehend aus einer elektronenziehenden Gruppe, Alkyl und Alkoxy, wobei die elektronenziehende Gruppe aus der Gruppe ausgewählt ist bestehend aus Fluor, Cyan, Perfluoralkyl, Nitro, -SO₂R und Kombinationen davon, wobei R Alkyl oder Perfluoralkyl ist.

6. Leuchtendes Material nach Anspruch 1, wobei R³ = R⁵ = H und R⁴ = R⁶ und aus der Gruppe ausgewählt ist bestehend aus verzweigtem Alkyl und Cycloalkyl.

7. Leuchtendes Material nach Anspruch 1, wobei R⁴ = R⁵ = H und R³ = R⁶ und aus der Gruppe ausgewählt ist bestehend aus verzweigtem Alkyl und Cycloalkyl.

8. Blauleuchtendes Material ausgewählt aus der Gruppe bestehend aus
Verbindung B1 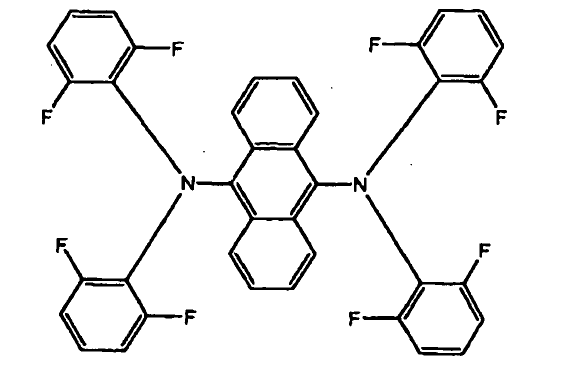 Verbindung B2 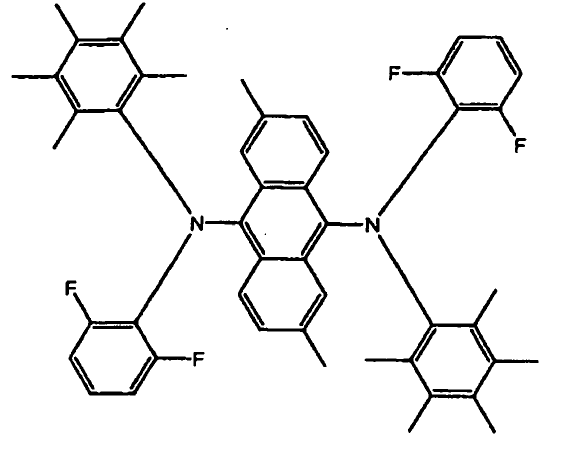 Verbindung B3 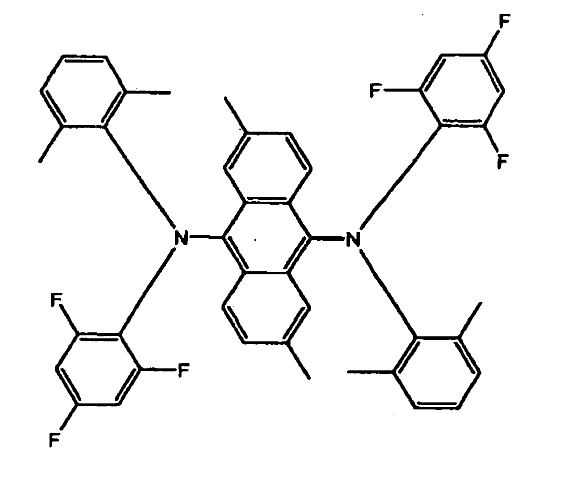 Verbindung B4 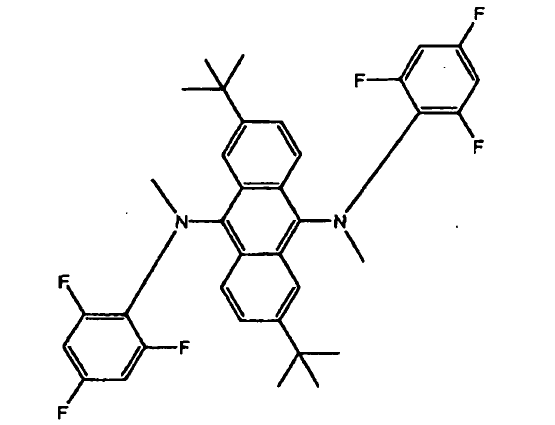 Verbindung B5 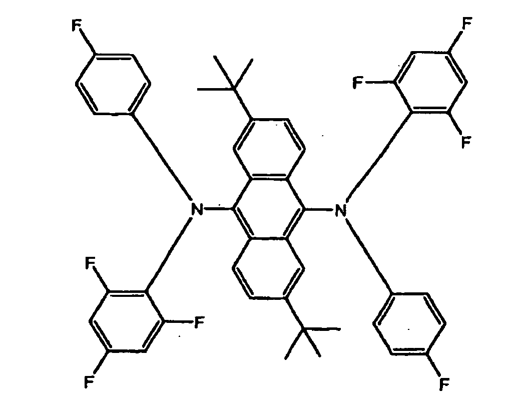 Verbindung B6 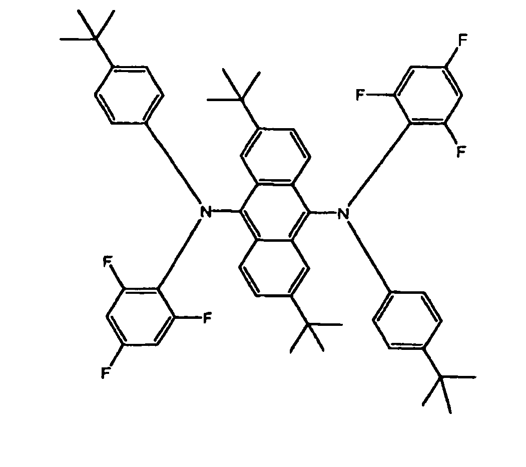 Verbindung B7 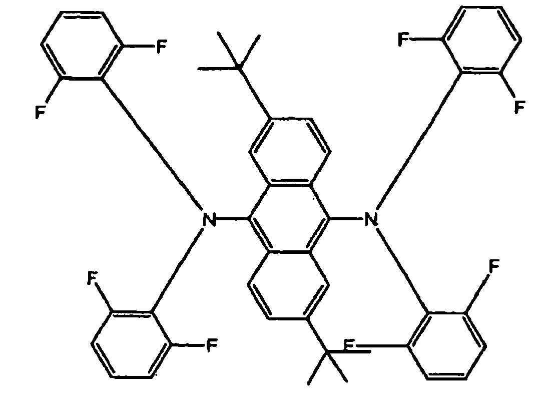 und Verbindung B8

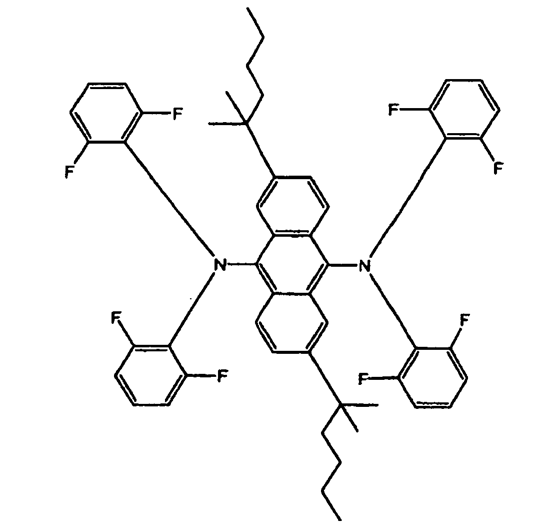

9. Organische elektronische Vorrichtung umfassend einen ersten elektrischen Kontakt, einen zweiten elektrischen Kontakt und eine photoaktive Schicht dazwischen, wobei die photoaktive Schicht ein blauleuchtendes Material umfasst, das die Formel I 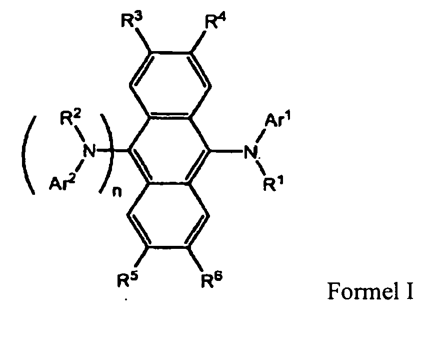 aufweist, wobei:
Ar1 und Ar2 gleich oder verschieden sind und jedes eine Arylgruppe ist, die mindestens zwei elektronenziehende Substituenten aufweist ausgewählt aus der Gruppe bestehend aus Fluor, Cyan, Perfluoralkyl, Nitro, -SO₂R und Kombinationen davon, wobei R Alkyl oder Perfluoralkyl ist;
R¹ und R² gleich oder verschieden und aus der Gruppe ausgewählt sind bestehend aus Alkyl und Aryl;
R³, R⁴, R⁵ und R⁶ gleich oder verschieden und aus der Gruppe ausgewählt sind bestehend aus Wasserstoff, Alkyl und Alkoxy; und
n 1 beträgt.

## Revendications

1. Matériau luminescent bleu de formule 1 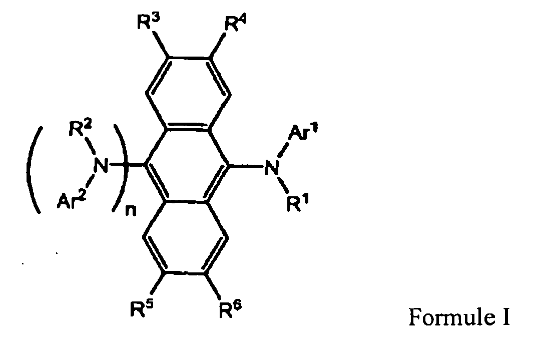 dans laquelle:
Ar1 et Ar2 sont identiques ou différents et chacun est un groupe aryle ayant au moins deux substituants électro-attracteurs choisis dans le groupe constitué du fluor et des groupes cyano, perfluoroalkyle, nitro, -SO₂R, et leurs combinaisons, R étant un groupe alkyle ou perfluoroalkyle;
R¹ et R² sont identiques ou différentes et sont choisis dans le groupe constitué des groupes alkyle et aryle;
R³, R⁴, R⁵ et R⁶ sont identiques ou différents et sont choisis dans le groupe constitué de l'hydrogène, et des groupes alkyle et alcoxy; et
n vaut 1.

2. Matériau luminescent selon la revendication 1, dans lequel Ar1 et Ar2 sont choisis dans le groupe constitué des groupes phényle, biphényle, naphtyle, et binaphtyle.

3. Matériau luminescent selon la revendication 1, dans lequel au moins un des R¹ et R² est un groupe alkyle.

4. Matériau luminescent selon la revendication 1, dans lequel R¹ et R² sont chacun des groupes aryle choisis dans le groupe constitué des groupes phényle, biphényle, napthyle, et binaphtyle.

5. Matériau luminescent selon la revendication 4, dans lequel le groupe aryle a au moins un substituant choisi dans le groupe constitué d'un groupe électro-attracteur, d'un groupe alkyle et d'un groupe alcoxy, le groupe électro-attracteur étant choisi dans le groupe constitué du fluor et des groupes cyano, perfluoroalkyle, nitro, -SO₂R, et leurs combinaisons, R étant un groupe alkyle ou perfluoroalkyle.

6. Matériau luminescent selon la revendication 1, dans lequel R³ = R⁵ = H, et R⁴ = R⁶ et est choisi dans le groupe constitué des groupes alkyle ramifiés et cycloalkyle.

7. Matériau luminescent selon la revendication 1, dans lequel R⁴ = R⁵ = H, et R³ = R⁶ et est choisi dans le groupe constitué des groupes alkyle ramifiés et cycloalkyle.

8. Matériau luminescent bleu choisi dans le groupe constitué des
Composé B1 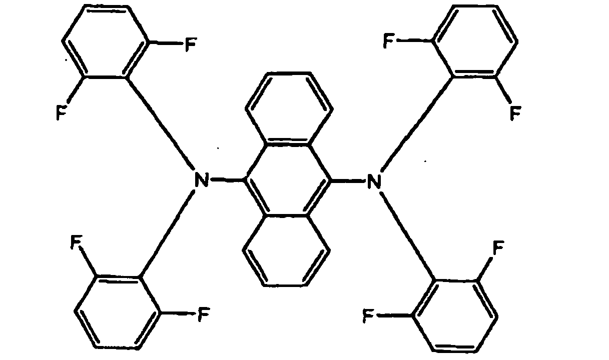 Composé B2 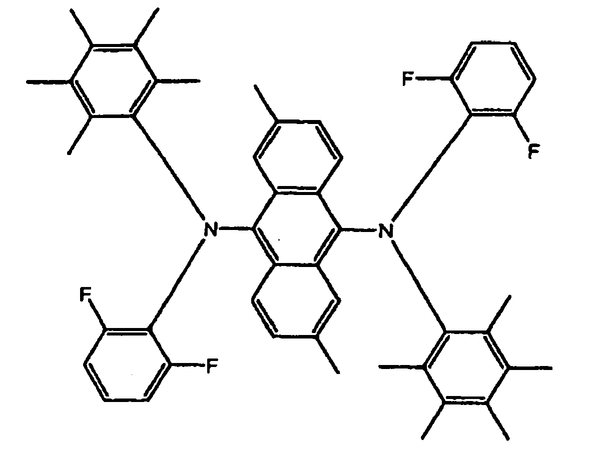 Composé B3 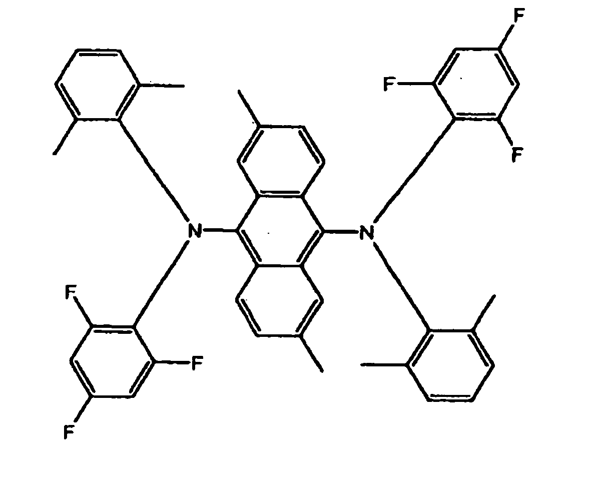 Composé B4 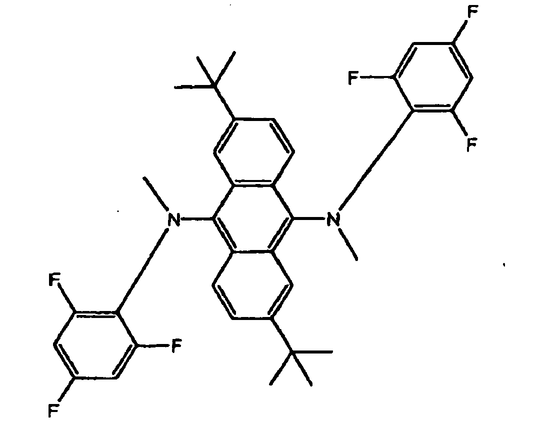 Composé B5 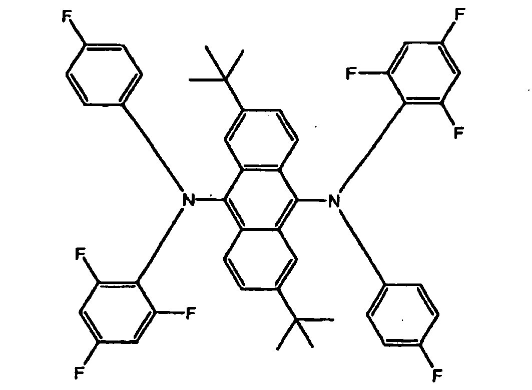 Composé B6 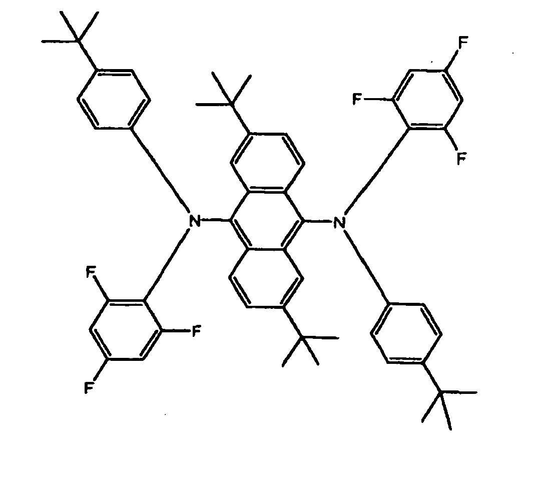 Composé B7 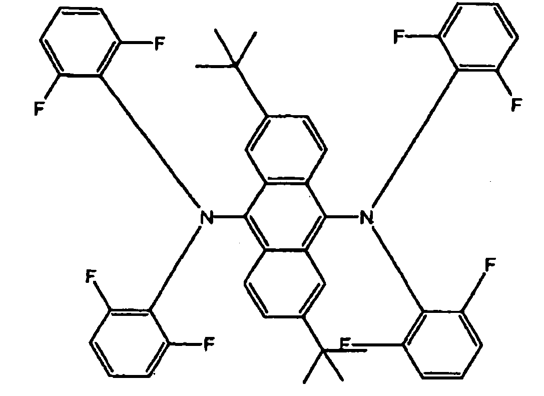 et Composé B8

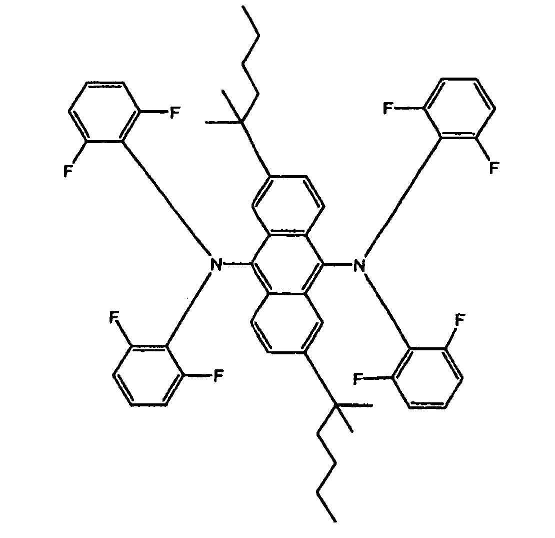

9. Dispositif électronique organique comprenant un premier contact électrique, un second contact électrique et une couche photoactive entre les deux, la couche photoactive comprenant un matériau luminescent bleu de formule 1 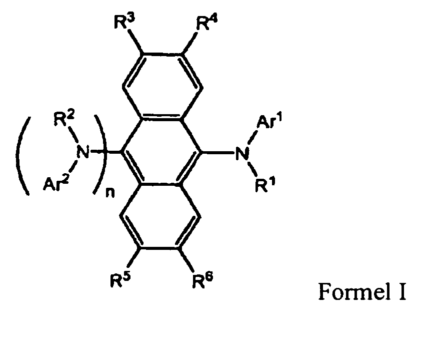 dans laquelle:
Ar1 et Ar2 sont identiques ou différents et chacun est un groupe aryle ayant au moins deux substituants électro-attracteurs choisis dans le groupe constitué du fluor et des groupes cyano, perfluoroalkyle, nitro, -SO₂R, et leurs combinaisons, R étant un groupe alkyle ou perfluoroalkyle;
R¹ et R² sont identiques ou différentes et sont choisis dans le groupe constitué des groupes alkyle et aryle;
R³, R⁴, R⁵ et R⁶ sont identiques ou différents et sont choisis dans le groupe constitué de l'hydrogène, et des groupes alkyle et alcoxy; et
n vaut 1.
